# EUROPÄISCHE PATENTSCHRIFT

(11) **EP 3 752 128 B1**
(45) Veröffentlichungstag und Bekanntmachung des Hinweises auf die Patenterteilung: **15.01.2025**
(21) Anmeldenummer: 19706526.1
(22) Anmeldetag: 18.02.2019
(51) Int. Cl.: A61K 31/7088, A61K 38/48, A61P 21/02, A61K 48/00, A61K 8/60, A61Q 19/08

(54) **NUKLEINSÄURE-BASIERTES BOTULINUM NEUROTOXIN ZUR THERAPEUTISCHEN ANWENDUNG**
NUCLEIC ACID-BASED BOTULINUM NEUROTOXIN FOR THERAPEUTIC USE
NEUROTOXINE BOTULIQUE REPOSANT SUR UN ACIDE NUCLÉIQUE, POUR APPLICATION THÉRAPEUTIQUE

(30) Priorität: 16.02.2018 DE 102018103504
(43) Veröffentlichungstag der Anmeldung: 23.12.2020
(73) Patentinhaber: preclinics discovery GmbH, 14482 Potsdam (DE)
(72) Erfinder: FÜNER, Jonas, 14473 Potsdam (DE)
(74) Vertreter: Hertin und Partner Rechts- und Patentanwälte PartG mbB
(86) Internationale Anmeldenummer: PCT/EP2019/053937
(87) Internationale Veröffentlichungsnummer: WO 2019/158745

(56) Entgegenhaltungen:
- WO-A1-2014/152121
- WO-A1-2016/131052
- WO-A1-2017/220800
- WO-A2-2004/071538
- WO-A2-2006/076902
- US-A1- 2002 107 199

## Beschreibung

Die Erfindung bezieht sich auf eine Nukleinsäure zur therapeutischen Anwendung die Botulinum Neurotoxin kodiert. Die Erfindung betrifft außerdem die Transfektion von Skelettmuskelzellen und glatten Muskelzellen und der Drüsen der Haut, sowie anderer Hautzellen mit Botulinum Neurotoxin (BoNT) kodierenden Nukleinsäuren (RNA oder DNA) mit oder ohne einem sekretorischen Signal für therapeutische und/oder kosmetische Zwecke.

### Stand der Technik

Botulinum Neurotoxine (BoNT) werden von den sporenbildenden Bakterien *Clostridium botulinum* in mindestens 7 Serotypen und mindestens 30 Subtypen produziert. Die Expression erfolgt aus einem charakteristischen Gencluster gemeinsam mit einer Reihe von Komplexproteinen, mehrere Hemagglutinine und ein non-toxin-non hemagglutinin Molekül von ähnlicher Größe wie das Neurotoxin selbst. Neurotoxin Serotyp A (als Beispiel) hat ein Molekulargewicht von 150kDa und besteht aus 2 Domänen, der heavy chain (100kDa) und der light chain (50kDa) die nach der Aktivierung noch durch eine Disulfidbrückenbindung verbunden sind. Die heavy chain Domäne besteht wiederum N-terminal aus der Translokationsdomäne durch die die light chain aus dem Vesikel in das Zytosol befördert wird. C-terminal enthält sie die Bindungsdomäne die an präsynaptische Ganglioside und an das vesikuläre SV2 Protein bindet. Die light chain entwickelt nach der Translokation in das Zytosol katalytische Aktivität und schneidet spezifisch das SNARE Protein SNAP-25 hinter Glutamin¹⁹⁷. Dadurch verliert der SNARE Komplex an Dichte und kann das Vesikel mit dem Neurotransmitter nicht mehr ausreichend fest an die Membraninnenseite des präsynaptischen Terminals ziehen, so dass es nicht mehr zu einer Fusion mit der Zellmembran und entsprechenden Freisetzung des gespeicherten Neurotransmitters in den synaptischen Spalt kommt. Die physiologische Folge dieser chemischen Denervierung ist postsynaptisch eine Muskelparalyse. Der Wirkmechanismus der Botulinum Neurotoxine umfasst 4 Schritte:
1. Die Bindung der heavy chain and Gt1b und SV2 am präsynaptischen Terminal bzw. am exponierten Freisetzungsvesikel,
2. Die Aufnahme in das Freisetzungsvesikel bei dessen Retraktion in das präsynaptische Terminal,
3. Die Translokation der light chain aus dem Vesikel in das präsynaptische Zytosol und
4. Die katalytische Prozessierung von SNAP-25 durch die light chain im Zytosol.

Die aus dem gleichen Gencluster exprimierten Komplexproteine bilden mit dem Neurotoxin verschiedene Komplexe, die das Neurotoxin gegen die niedrigen pH-Werte im Magen und proteolytische Enzyme schützen bis der Komplex im Duodenum bei alkalischen pH-Werten dissoziiert und das Neurotoxin wahrscheinlich wieder mithilfe der Hemagglutine parazellulär durch die Mukosa in das Blut aufgenommen wird. Dieser Mechanismus spielt nur bei der natürlich vorkommenden Funktion des Neurotoxins als Fraßgift eine Rolle, während es bei den therapeutischen Verwendungen in der Medizin intramuskulär oder intraglandulär injiziert wird, wobei die Toxinwirkung auch ohne die Komplexproteine funktioniert.

Botulinum Neurotoxin ist bereits seit langem als Arzneimittel zugelassen (z.B. BOTOX, Xeomin, Dysport, Myobloc).

Das Botulinum Neurotoxin (BoNT) ist besonders bekannt für seinen Einsatz in der ästhetischen Medizin, weniger bekannt ist die außerordentlich wichtige Bedeutung für die Behandlung von Spastiken und Dystonien, sowie der Verringerung der Hyperhidrose oder von Speichelauslaufen, das i.d.R. durch verminderte Schluckfähigkeit bedingt ist (z.B. bei Morbus Parkinson, amyotropher Lateralsklerose, infantiler Cerebralparese).

Die Wirkung beruht auf der Hemmung der Erregungsübertragung am präsynaptischen Teil der motorischen Endplatte und der damit einhergehenden Paralyse der Muskelzelle oder, im Falle der Speicheldrüsen, der autonomen Innervation der Drüsenzellen.

Auf der anderen Seite ist BoNT eines der stärksten Toxine und eine Dosis von weniger als 1 mg kann bereits zum Tod eines Menschen führen.

Abhängig von der verwendeten Dosis und des verwendeten BoNT Serotyps variiert die Dauer der Wirkung. In der Regel wird BoNT/A verwendet, es gibt aber auch BoNT/B Präparate z.B. Neurobloc mit einer kürzeren Wirkdauer. BoNT/E Produkte befinden sich derzeit noch nicht auf den Markt, es gibt jedoch schon fortgeschrittene Entwicklungen für ein solches Produkt.

Bei den chronischen Erkrankungen wird eine besonders lange Wirkdauer angestrebt, da die Behandlungsprozedur für den Patienten unangenehm und mit Schmerzen verbunden ist. Außerdem sind die Behandlungskosten recht hoch, da die Behandlung nur von Ärzten mit spezieller Erfahrung durchgeführt werden kann. Weiterhin erhöht sich mit der Dosis und der Anzahl der Behandlungen die Gefahr, dass der Patient neutralisierende Antikörper gegen BoNT entwickelt und dadurch zum sekundären Nonresponder wird, für den diese wertvolle und oft einzige Therapie dann nicht mehr zur Verfügung steht.

Um eine lange Wirkdauer zu erhalten, ist es notwendig eine möglichst hohe Dosis zu verabreichen. Jedoch wird bei dem bisherigen therapeutischen Verfahren, der Applikation von BoNT in Lösung, mit einer Erhöhung der lokalen Dosis auch die systemische Dosis erhöht. Die Erhöhung der systemischen Dosis kann zu gravierenden Nebenwirkungen führen und ist daher riskant. In der Regel ist die Wirkdauer von injiziertem BoNT/A Protein auf ca. 3-4 Monate beschränkt.

Die Produktion von BoNT-Arzneimitteln erfolgt entweder in Clostridien oder rekombinant in einem geeigneten Expressionssystem wie z.B. E.coli. Für beides werden entsprechende Sicherheitsbereiche benötigt und es müssen große Mengen von Toxin gehandhabt werden. Dies ist auch ein gesellschaftliches Risiko, da bei dem Vorhandensein großer Toxinmengen auch immer die Gefahr der Entwendung und der missbräuchlichen Verwendung besteht. Schließlich gehört BoNT zu der Kategorie A der biologischen Kampfstoffe entsprechend der Einordnung des Centers for Disease Control (USA). Komplizierend ist weiterhin, dass auch die hochregulierte, pharmazeutisch-industrielle Herstellung biologischen Schwankungen unterliegt und somit für jede Charge eine Aktivitätskontrolle durchgeführt bzw. die Zubereitungen biologisch standardisiert werden müssen. Eine "Einheit" entspricht dabei der Menge an Botulinumtoxin, die nach intraperitonealer Gabe bei Mäusen tödlich wirkt und zum Tod von 50% der behandelten Tiere führt (LD50). Hier wird also ein Test angewandt, der in anderen Bereichen der Toxikologie weitgehend abgeschafft wurde. Es sind zwar in jüngster Zeit zellbasierte alternative Verfahren entwickelt worden, aber derzeit werden noch viele Tierversuche für diese Tests durchgeführt.

Ein Nachteil ist, dass die therapeutische Breite von BoNT sehr gering ist; dies wird naturgemäß weniger bei ästhetischen Anwendungen wie Zornesfalten auf der Stirn als bei Hochdosisanwendungen wie Spastik nach Schlaganfall oder infantiler Zerebralparese bedeutsam. Durch die hohe Toxizität besteht immer die Gefahr der Überdosierung mit systemischen oder vom Injektionsort distanten Nebenwirkungen. Die Dauer der Wirkung ist zwar lange aber doch begrenzt. Da das Medikament lokal intramuskulär oder intraglandulär injiziert wird ist die Behandlung zudem unangenehm und mit Schmerzen verbunden.

Aufgrund der zahlreichen und unterschiedlichen Nachteile wäre es daher gut, wenn auf die Produktion des Toxins verzichtet werden könnte und es ein standardisiert herzustellendes Arzneimittel gäbe, von welchem keine Gefahr für Mensch und Umwelt ausgehen würde. Weiterhin wäre es vorteilhaft, die Gefahr der Überdosierung zu vermeiden oder systemische Nebenwirkungen stark zu reduzieren. Ebenso wäre es sehr vorteilhaft mehr Möglichkeiten bei der Einstellung der Wirkungsdauer und der Wirkungsstärke zu erhalten.

US 2002/107199 A1 beschreibt DNA welche Botulinum Neurotoxin codiert und genutzt wird zur direkten Transformation von Zellen und Behandlung von verschiedenen Organen und Krankheiten: Schmerz, Skelettmuskel, glatter Muskel, Drüsen, Spastiken, Dystonien, diverse Bewegungsstörungen und gestörte Muskelaktivität (Gliederspastik, Augenlid, Tics, Tremor, Bruxismus), Hyperhydrose, Strinfalten, Hautfalten etc. Das Botulinum Toxin kann jeder Form sein A, B, C1, D, E, F oder G. Als Beispiel werden Haut- und Muskelzellen mit BonNT/A transformiert und in menschlichen Patienten entzündliche Schmerzen sowie gustatorische Hyperhydrose (Frey's Syndrom) behandelt.

WO 2004/071538 A2 beschreibt Baculovirus-Konstrukte welche eine DNA codierend für ein Botulinum-Toxin enthalten. Sie mit sind Promotor und Sekretionssignal ausgestattet so dass das Toxin in den Keratinozyten Zielzellen exprimiert und sekretiert werden kann um das umliegende Skelett-Muskelgewebe zu relaxieren. Typischerweise wird dieses Konstrukt auf die Haut aufgetragen mittels Lotion oder Träger wo es dann seine Anti-Falten Wirkung entfaltet.

Die der Erfindung zugrunde liegende Aufgabe war es daher, ein alternatives Medikament für den bisherigen BoNT Wirkstoff bereitzustellen, das die Nachteile des Standes der Technik überwindet.

### Beschreibung der Erfindung

Gelöst wird die Aufgabe durch die unabhängigen Ansprüche. Bevorzugte Ausführungsformen befinden sich in den abhängigen Ansprüchen.

In einer ersten bevorzugten Ausführungsform betrifft die Erfindung eine Nukleinsäure zur Verwendung als Arzneimittel und/oder in therapeutischen Verfahren, die eine Botulinum Neurotoxin kodierende Nukleinsäure umfasst oder aus dieser besteht, wobei die BoNT kodierende Nukleinsäuresequenz so modifiziert ist, dass das kodierte Protein zwischen leichter und schwerer Kette im Linkerbereich ein Erkennungsmotiv für eine Protease, bevorzugt LVPRGS oder LVPRGS umfasst.

Die Erfindung betrifft also auch den Einsatz von BoNT Nukleinsäure zur Transfektion von Zellen, damit diese Zellen das BoNT Protein selbst herstellen und an den Wirkort transportieren.

Die Dosierung der Expression eines rekombinanten Produktes lässt sich nicht so genau dosieren, wie die direkte Injektion eines gelösten Proteins. Dies ist wahrscheinlich einer der Gründe, weshalb im Stand der Technik die rekombinante Therapievariante mittels in den Muskel oder in die Haut applizierter Nukleinsäuren noch nicht beschrieben wurde. Dass die Dosierungsgenauigkeit bei dem gentherapeutischen Verfahren aber weniger wichtig ist, weil die systemische Verfügbarkeit deutlich reduziert wird, ist eine wichtige Erkenntnis und somit zentraler Bestandteil der Erfindung.

Die Erfindung betrifft damit auch den erstmaligen Einsatz in der Gentherapie von einer hier beschriebenen Nukleinsäure die BoNT kodiert.

Ein Vorteil der Erfindung ist, dass durch die Applikation der Nukleinsäure eine längere Wirkdauer erzielt werden kann. Da die Behandlungsprozedur aus dem Stand der Technik (Gabe des Toxins selbst) für den Patienten unangenehm und mit Schmerzen verbunden ist, kann die Behandlung so deutlich angenehmer gestaltet werden.

Auch die Behandlungskosten können durch diesen neuen Ansatz perspektivisch gesenkt werden.

Die Nukleinsäure gemäß der Erfindung kann dann in die Zellen, z.B. Muskelzellen transfiziert werden. Das rekombinante BoNT Protein wird dann direkt in der Muskelzelle gebildet und gerät von dort an den Wirkort, den synaptischen Spalt.

Erfindungsgemäß können die Nukleinsäuren oder Nukleinsäuremoleküle auch in einem Vektor vorliegen.

Muskelzellen lassen sich besonders einfach transfizieren. Nach intramuskulärer oder intradermaler Applikation von nackter Plasmid-DNA, oder RNA mit einem entsprechenden Transfektionsvektor kann relativ einfach eine Expression des rekombinanten Proteins in dem Zielorgan erreicht werden (vgl. z. B. Davis, Whalen & Demeneix "Direct gene transfer into skeletal muscle in vivo: factors affecting efficiency of transfer and stability of expression" Hum Gene Ther. 1993 Apr;4(2):151-9; or Danko & Wolff, "Direct gene transfer into muscle." Vaccine. 1994 Dec; 12(16):1499-502).

In einer bevorzugten Ausführungsform ist die Nukleinsäure der Erfindung für die direkte Applikation in das zu behandelnde Organ und/oder Muskel hergerichtet.

In einer besonders bevorzugten Ausführungsform wird die Nukleinsäure als DNA oder RNA hergerichtet. Besonders bevorzugt ist der Einsatz von RNA, da hier bisher die besten Ergebnisse erzielt werden konnten.

Die Nukleinsäure der Erfindung kann auch einen Promotor enthalten. Es wurden aber auch Versuche mit mRNA durchgeführt, die keinen Promoter benötigten. Bei Versuchen mit Plasmid-DNA wurde ein CMV Promotor verwendet, der gut funktioniert. Es können aber auch andere Promotoren eingesetzt werden, sodass die Erfindung in diesem Punkt nicht beschränkt werden soll.

Durch eine Nukleinsäure basierte BoNT Therapie kann auf die Herstellung von BoNT im Labor als Pharmakon verzichtet werden. Es werden nur die Nukleinsäuren hergestellt. Das Protein selbst wird im Körper z.B. in den Muskelzellen des Patienten hergestellt.

Durch die Behandlung mit Nukleinsäuren und somit der Verabreichung eines rekombinanten BoNT in den Zellen am Zielort, kann nicht nur eine höhere lokale Dosierung erzielt werden, sondern diese kann auch über einen längeren Zeitraum die Dosis aufrechterhalten.

In einer weiteren bevorzugten Ausführungsform betrifft die Erfindung eine Nukleinsäure umfassend eine BoNT kodierende Nukleinsäure und ein S/MAR Element. S/MAR steht für scaffold/matrix attachment region (Kerngerüst-/Kernmatrixanheftungsregionen). Diese natürlich in allen Organismen vorkommenden DNA-Sequenzen sorgen für die Verankerung der DNA/des Chromatins an die Zellkernmatrix. Des Weiteren haben diese Elemente Einfluss auf die Expression von Genen, in dem sie mit Enhancer interagieren bzw. rekrutieren oder nicht und indem sie die Doppelhelixstruktur destabilisieren.

Es konnte gezeigt werden, dass S/MAR-Elemente die Expression von transfizierter DNA verlängern kann und die Transfektion mehrerer Zellen insgesamt homogener verläuft. Es wird vermutet, dass das mittransfizierte S/MAR-Element dafür sorgt, dass die transfizierte DNA nicht ins Wirtsgenom integriert wird, sondern selbst and die Kernmatrix bindet. Somit geht die DNA bei der Zellteilung nicht verloren, wird aber auch nicht an einer ungünstigen Stelle im Wirtsgenom integriert. Letzteres führt in der Regel zur Stilllegung der Expression des transfizierten Gens oder zu Nebenwirkungen auf die umliegenden Wirtsgene. Mittels S/MAR-Element können die negativen Aspekte umgangen werden.

Durch unterschiedliche Nukleinsäureformulierungen (mRNA oder plasmidDNA, verschiedene Promotoren, S/MAR usw.) kann eine kurze oder besonders lange Wirkdauer eingestellt werden. Der Fachmann ist in der Lage die geeignete Formulierung für die jeweilige Anwendung zu identifizieren, ohne dabei selbst erfinderisch tätig zu werden.

Durch die Freisetzung des Proteins direkt am Wirkort wird die Gefahr der Überdosierung stark reduziert und das immunogene Potential gesenkt.

In einer besonders bevorzugten Ausführungsform betrifft die Erfindung Botulinum Neurotoxin kodierenden Nukleinsäuren zur Behandlung von Spastiken, Dystonien, Speichelauslaufen und/oder der Hyperhydrose. Die Nukleinsäure der Erfindung kann daher auch bei Morbus Parkinson, amyotropher Lateralsklerose oder infantiler Cerebralparese zum Einsatz kommen, da diese Krankheitsbilder mit verminderter Schluckfähigkeit einhergehen können und somit z.B. zu Speichelauslaufen führen können.

Die Nukleinsäuren der Erfindung können aber auch bei anderen Erkrankungen eingesetzt werden, die mit Bewegungsstörungen oder gestörter Muskelaktivität einhergehen.

Es war völlig überraschend, dass die Erfindung mit und ohne sekretorischem Signal erfolgreich umgesetzt werden kann. Die Transfektion der Skelettmuskelzelle ohne sekretorischem Signal führt zu einer intrazellulären Expression des BoNT. Anders als zu erwarten, gelangt das BoNT aus dem Zytoplasma in den synaptischen Spalt der motorischen Endplatte und somit an den Wirkort, die präsynaptische cholinerge Nervenendigung. Auf diese Weise wird eine toxische systemische Wirkung vermieden. Nachteilig ist bei dieser Methode, dass nur die Nervenendigungen in der Nähe der transfizierten Zellen paralysiert werden und somit die gleichmäßige Verteilung der Transfektion eine Voraussetzung für den Erfolg ist. Dieser Nachteil kann über die Verwendung eines sekretorischen Signals vermieden werden, nichtdestotrotz ist es bemerkenswert, dass BoNT auch ohne dieses Signal an den Wirkort gelangt. Je nach Indikation, kann diese Ausführungsform durchaus vorteilhaft sein.

Bei der Verwendung des sekretorischen Signals gelangt zwar das BoNT auch außerhalb der neuromuskulären Synapse in den extrazellulären Raum, aber die systemische Verteilung ist je nach Dosierung deutlich geringer oder überhaupt nicht vorhanden. Bei einer normalen Applikation des BoNT Proteins gemäß dem Stand der Technik wird die Flüssigkeit der BoNT-Lösung schnell resorbiert und bei dieser Resorption gelingt auch BoNT in die Zirkulation. Da bei der Transfektion die Applikation der Nukleinsäuren und die Expression des BoNT zeitlich entkoppelt ist, hat sich das Gewebe um die Applikationsstelle zum Zeitpunkt der Sekretion wieder normalisiert. Es ist keine vermehrte Flüssigkeit in dem extrazellulären Raum und das BoNT wandert entlang der Muskelmembran zu den motorischen Endplatten, also an den Wirkort.

Als sekretorische Signal kann beispielsweise ein Signalpeptid von murinem IgG (Kappa) verwendet werden. Die Signalsequenz wird dabei bevorzugt vor dem Luziferase-Gen eingefügt (N-terminal). Die Sequenz lautet (SEQ ID NO 6):

ATGGAGACCGACACCCTGCTGCTGTGGGTGCTGCTGCTGTGGGTGCCCGGCAGCACC
GGCGAC

Dabei handelt es sich jedoch nur um ein Beispiel. Es können unterschiedliche sekretorische Signale zum Einsatz kommen. Die Wahl hängt dabei auch vom Einsatzgebiet, also der zu transfizierenden Zelle und der Indikation ab.

Die Erfindung kann demnach mit oder ohne sekretorischem Signal eingesetzt werden. Bei der Verwendung der entsprechenden Sequenz ohne sekretorischem Signal war es bereits völlig überraschend, dass das BoNT aus dem Zytoplasma einer Muskelzelle in den synaptischen Spalt der motorischen Endplatte gelangen kann. Bei der Ausführungsform, welche ein sekretorisches Signal beinhaltet, liegt der entscheidende Vorteil gegenüber dem Stand der Technik vor allem in der niedrigeren systemischen Verteilung bei gleicher oder höherer Dosierung im Vergleich zu dem Stand der Technik.

Neben der weitgehenden Vermeidung von unerwünschten systemischen Wirkungen, wird auch die Gefahr der Bildung von Antikörpern gegen BoNT durch den Patienten deutlich reduziert, da das BoNT nur in geringem Maße extrazellulär präsent ist. Es befindet sich größtenteils entweder in der transfizierten Zelle, welche das BoNT produziert, oder bereits in der Zielzelle der efferenten Nervenfaser.

Ein weiterer Vorteil ist die einfachere und preisgünstigere Produktion. In der Herstellung kann man einen deutlichen Kostenvorteil erzielen, da diese standardisierter ist.

Die aufwendige und umstrittene Aktivitätsbestimmung entfällt bei diesem rekombinanten Produkt, was einen erheblichen Vorteil gegenüber dem Stand der Technik darstellt. Denn Tierversuche für die Chargenfreigabe wären für die Produktion des erfindungsgemäßen Stoffes nicht mehr notwendig.

Für die Behandlung der Patienten ergeben sich völlig neue Optionen. Durch die Wahl des Transfektionsvektors kann die Dauer der Expression gesteuert werden und außerdem kann der Anteil der transfizierten Zellen variiert werden. Hierdurch können die Wirkungsdauer und die Wirkungsstärke beeinflusst werden. Diese Flexibilität ist ein entscheidender Vorteil für die therapeutische Anwendung, da diese nun zielgenauer an den jeweiligen Patienten angepasst werden kann.

Für einige therapeutische Anwendung ist z.B. eine partielle Paralyse vorteilhaft, wie z.B. bei der Osteosynthese, bei welcher es vorteilhaft wäre, den Muskeltonus des umliegenden Gewebes für die erste Heilungsphase zu senken. Solche Anwendungen sind durch die Erfindung nun erstmals möglich.

Durch die reduzierte systemische Verfügbarkeit, sinkt nicht nur die Toxizität, sondern auch die Immunogenität des BoNT. Dies ist ein großer Vorteil für Patienten mit chronischen Erkrankungen, welche auf eine kontinuierliche Therapie angewiesen sind.

Alle bekannten Serotypen von BoNT, bzw. die entsprechenden Nukleinsäuren, können für die Erfindung eingesetzt werden. D.h. die Erfindung betrifft vor allem Nukleinsäuren zur Kodierung von BoNT/A, BoNT/B, BoNT/C, BoNT/D, BoNT/E, BoNT/F und BoNT/G. Bevorzugt sind dabei Nukleinsäuren zur Kodierung von BoNT/A, BoNT/B, BoNT/E und BoNT/F. Besonders bevorzugt sind Nukleinsäuren zur Kodierung von BoNT/A, BoNT/B und BoNT/E.

Neben dem Einsatz unterschiedlicher Transfektionsvektoren kann die Gen-Sequenz der natürlich vorkommenden BoNT-Serotypen modifiziert werden und dadurch veränderte Eigenschaften des Proteins erreicht werden, wie beispielsweise Veränderung der Wirkdauer, eine andere Targetzellpopulation, ein anderer Effekt der translozierten leichten Kette oder eine stärkere Ausschleusung aus den Muskelzellen. Eine verstärkte Ausschleusung aus der Muskelzelle, kann z.B. durch die Insertion eines sekretorischen Signals erfolgen.

Die zur Aktivierung des exprimierten Neurotoxins erforderliche Protease kann zusätzlich exprimiert werden oder diese kann nach der *in vivo* Transfektion durch lokale Injektion einer geringen Dosis eingebracht werden. Als solche Proteasen kommen z.B. Thrombin oder Stuart-Prower-Faktor/Faktor Xa in Betracht, die als Arzneimittelfertigprodukte (z.B. Recothrom^{®}, Coagadex^{®}) verfügbar sind. In die BoNT Sequenz wird zu diesem Zweck entsprechend zwischen leichter und schwerer Kette im Linkerbereich ein Erkennungsmotiv für die jeweilige Protease (Thrombin: LVPRGS, Faktor: Xa LVPRGS) eingefügt. Diese Vorgehensweise führt zur weiteren Fokussierung des BoNT Effektes auf das injizierte Areal oder Muskelkompartiment und vermeidet somit systemische oder entfernte unerwünschte Nebenwirkungen des Neurotoxins.

Für diese Ausführungsform eignet sich besonders eine Nukleinsäure die eine Sequenz umfasst die für SEQ ID NO 4 kodiert.

Auch bevorzugt ist eine Codon-Optimierung der BoNT Sequenz für die Expression in humanen Zellen wodurch die Effizienz der Expression verbessert werden kann.

Bevorzugt ist, dass die Nukleinsäure eine Sequenz umfasst, die eine der folgenden Proteinsequenzen kodiert:
SEQ ID No 1 (BoNT/A Hall Strain): 
MPFVNKQFNYKDPVNGVDIAYIKIPNAGQMQPVKAFKIHNKIWVIPERDTFTNPEEGDLNPPPEAKQVP
VSYYDSTYLSTDNEKDNYLKGVTKLFERIYSTDLGRMLLTSIVRGIPFWGGSTIDTELKVIDTNCINVIQPDG
SYRSEELNLVIIGPSADIIQFECKSFGHEVLNLTRNGYGSTQYIRFSPDFTFGFEESLEVDTNPLLGAGKFATD
PAVTLAHELIHAGHRLYGIAINPNRVFKVNTNAYYEMSGLEVSFEELRTFGGHDAKFIDSLQENEFRLYYYN
KFKDIASTLNKAKSIVGTTASLQYMKNVFKEKYLLSEDTSGKFSVDKLKFDKLYKMLTEIYTEDNFVKFFKVL
NRKTYLNFDKAVFKINIVPKVNYTIYDGFNLRNTNLAANFNGQNTEINNMNFTKLKNFTGLFEFYKLLCVR
GIITSKTKSLDKGYNKALNDLCIKVNNWDLFFSPSEDNFTNDLNKGEEITSDTNIEAAEENISLDLIQQYYLTF
NFDNEPENISIENLSSDIIGQLELMPNIERFPNGKKYELDKYTMFHYLRAQEFEHGKSRIALTNSVNEALLNP
SRVYTFFSSDYVKKVNKATEAAMFLGWVEQLVYDFTDETSEVSTTDKIADITIIIPYIGPALNIGNMLYKDDF
VGALIFSGAVILLEFIPEIAIPVLGTFALVSYIANKVLTVQTIDNALSKRNEKWDEVYKYIVTNWLAKVNTQID
LIRKKMKEALENQAEATKAIINYQYNQYTEEEKNNINFNIDDLSSKLNESINKAMININKFLNQCSVSYLMN
SMIPYGVKRLEDFDASLKDALLKYIYDNRGTLIGQVDRLKDKVNNTLSTDIPFQLSKYVDNQRLLSTFTEYIK
NIINTSILNLRYESNHLIDLSRYASKINIGSKVNFDPIDKNQIQLFNLESSKIEVILKNAIVYNSMYENFSTSFWI
RIPKYFNSISLNNEYTIINCMENNSGWKVSLNYGEIIWTLQDTQEIKQRVVFKYSQMINISDYINRWIFVTIT
NNRLNNSKIYINGRLIDQKPISNLGNIHASNNIMFKLDGCRDTHRYIWIKYFNLFDKELNEKEIKDLYDNQS
NSGILKDFWGDYLQYDKPYYMLNLYDPNKYVDVNNVGIRGYMYLKGPRGSVMTTNIYLNSSLYRGTKFII
KKYASGNKDNIVRNNDRVYINVVVKNKEYRLATNASQAGVEKILSALEIPDVGNLSQVVVMKSKNDQGIT
NKCKMNLQDNNGNDIGFIGFHQFNNIAKLVASNWYNRQIERSSRTLGCSWEFIPVDDGWGERPL
SEQ ID No. 2 (BoNT/E): 
MPKINSFNYNDPVNDKTILYIKPGGCQQFYKSFNIMKNIWIIPERNVIGTIPQDFLPPTSLKNGDSSYYDPN
YLQSNEEKDRFLKIVTKIFNRINDNLSGRILLEELSKANPYLGNDNTPDNQFHIGDASAVEIKFSNGNQSILL
PNVIIMGAEPDLFETNSSNISLRNNYMPSNHGFGSIAIVTFSPEYSFRFNDNSMNEFIQDPALTLMHELIHS
LHGLYGAKRITTKYTITQQQNPLITNIRGTNIEEFLTFGGTDLNIITSAQYNDIYTNLLADYKKIASKLSKVQVS
 
NPQLNPYKDIFQEKYGLDKNASGIYSVNINKFDDIFKKLYSFTEFDLATKFQVKCRQTYIGQYKYFKLSNLLN
NSIYNISEGYNINTLKVNFRGQNTNLNPRIITQLTGRGLVKKIIRFCKNIVFSKGITKSICIEINNGELFFVASEN
SYNDDNINTPKEIDDTVTSNNNYENDLDQVILNFNSESAPGLSDEKLNLTIQNDAYIPKYDSNGTSDIEQH
DVNELNVFFYLDAQKVPEGENNVNLTSSIDTALLEQPKIYTFFSSEFINNVNKPVQAALFVSWIQQVLVDF
TTEANQKSTVDKIADISIVVPYIGLALNIGNEAQKGNFKDALELLGAGILLEFEPELLIPTILVFTIKSFLGSSDN
KNKVIKAINNALKERDEKWKEVYSFIVSNWMTKINTQFNKRKEQMYQALQNQVNAIKTIIESKYNSYTLEE
KNELTNKYDIKQIENELNQKVSIAMNNIDRFLTESSISYLMKLINEVKINKLREYDENVKTYLLNYIIQHGSIL
GESQQELNSMVTDTLNNSIPFKLSSYTDDKILISYFNKFFKRIKSSSVLNMRYKNDKYVDTSGYDSNINING
DVYKYPTNKNQFGIYNDKLSEVNISQNDYIIYDNKYKNFSISFWVRIPNYDNKIVNVNNEYTIINCMRDNN
SGWKVSLNHNEIIWTLQDNAGINQKLAFNYGNANGISDYINKWIFVTITNDRLGDSKLYINGNLIDQKSIL
NLGNIHVSDNILFKIVNCSYTRYIGIRYFNIFDKELDETEIQTLYSNEPNTNILKDFWGNYLLYDKEYYLLNVLK
PNNFIDRRKDSTLSINNIRSTILLANRLYSGIKVKIQRVNNSSTNDNLVRKNDQVYINFVASKTHLFPLYADT
ATTNKEKTIKISSSGNRFNQVVVMNSVGNNCTMNFKNNNGNNIGLLGFKADTVVASTWYYTHMRDHT
NSNGCFWNFISEEHGWQEK
SEQ ID No 3 (BoNT/B): 
MPVTINNFNYNDPIDNDNIIMMEPPFARGTGRYYKAFKITDRIWIIPERYTFGYKPEDFNKSSGIFNRDVC
EYYDPDYLNTNDKKNIFFQTLIKLFNRIKSKPLGEKLLEMIINGIPYLGDRRVPLEEFNTNIASVTVNKLISNP
GEVERKKGIFANLIIFGPGPVLNENETIDIGIQNHFASREGFGGIMQMKFCPEYVSVFNNVQENKGASIFN
RRGYFSDPALILMHELIHVLHGLYGIKVDDLPIVPNEKKFFMQSTDTIQAEELYTFGGQDPSIISPSTDKSIYD
KVLQNFRGIVDRLNKVLVCISDPNININIYKNKFKDKYKFVEDSEGKYSIDVESFNKLYKSLMLGFTEINIAEN
YKIKTRASYFSDSLPPVKIKNLLDNEIYTIEEGFNISDKNMGKEYRGQNKAINKQAYEEISKEHLAVYKIQMC
KSVKVPGICIDVDNENLFFIADKNSFSDDLSKNERVEYNTQNNYIGNDFPINELILDTDLISKIELPSENTESLT
DFNVDVPVYEKQPAIKKVFTDENTIFQYLYSQTFPLNIRDISLTSSFDDALLVSSKVYSFFSMDYIKTANKVV
EAGLFAGWVKQIVDDFVIEANKSSTMDKIADISLIVPYIGLALNVGDETAKGNFESAFEIAGSSILLEFIPELLI
PVVGVFLLESYIDNKNKIIKTIDNALTKRVEKWIDMYGLIVAQWLSTVNTQFYTIKEGMYKALNYQAQALE
EIIKYKYNIYSEEEKSNININFNDINSKLNDGINQAMDNINDFINECSVSYLMKKMIPLAVKKLLDFDNTLKK
NLLNYIDENKLYLIGSVEDEKSKVDKYLKTIIPFDLSTYSNIEILIKIFNKYNSEILNNIILNLRYRDNNLIDLSGYG
AKVEVYDGVKLNDKNQFKLTSSADSKIRVTQNQNIIFNSMFLDFSVSFWIRIPKYRNDDIQNYIHNEYTIIN
CMKNNSGWKISIRGNRIIWTLIDINGKTKSVFFEYNIREDISEYINRWFFVTITNNLDNAKIYINGTLESNMD
IKDIGEVIVNGEITFKLDGDVDRTQFIWMKYFSIFNTQLNQSNIKEIYKIQSYSEYLKDFWGNPLMYNKEYY
MFNAGNKNSYIKLVKDSSVGEILIRSKYNQNSNYINYRNLYIGEKFIIRRESNSQSINDDIVRKEDYIHLDLVL
HHEEWRVYAYKYFKEQEEKLFLSIISDSNEFYKTIEIKEYDEQPSYSCQLLFKKDEESTDDIGLIGIHRFYESGV
LRKKYKDYFCISKWYLKEVKRKPYKSNLGCNWQFIPKDEGWTE
SEQ ID NO 4 (BoNT/A Hall Strain mit Proteaseerkennungsmotiv):

MPFVNKQFNYKDPVNGVDIAYIKIPNAGQMQPVKAFKIHNKIWVIPERDTFTNPEEGDLNPPPEAKQVP
VSYYDSTYLSTDNEKDNYLKGVTKLFERIYSTDLGRMLLTSIVRGIPFWGGSTIDTELKVIDTNCINVIQPDG
SYRSEELNLVIIGPSADIIQFECKSFGHEVLNLTRNGYGSTQYIRFSPDFTFGFEESLEVDTNPLLGAGKFATD
PAVTLAHELIHAGHRLYGIAINPNRVFKVNTNAYYEMSGLEVSFEELRTFGGHDAKFIDSLQENEFRLYYYN

KFKDIASTLNKAKSIVGTTASLQYMKNVFKEKYLLSEDTSGKFSVDKLKFDKLYKMLTEIYTEDNFVKFFKVL
NRKTYLNFDKAVFKINIVPKVNYTIYDGFNLRNTNLAANFNGQNTEINNMNFTKLKNFTGLFEFYKLLCVR
GIITSKTKSLVPRGSKALNDLCIKVNNWDLFFSPSEDNFTNDLNKGEEITSDTNIEAAEENISLDLIQQYYLTF
NFDNEPENISIENLSSDIIGQLELMPNIERFPNGKKYELDKYTMFHYLRAQEFEHGKSRIALTNSVNEALLNP
SRVYTFFSSDYVKKVNKATEAAMFLGWVEQLVYDFTDETSEVSTTDKIADITIIIPYIGPALNIGNMLYKDDF
VGALIFSGAVILLEFIPEIAIPVLGTFALVSYIANKVLTVQTIDNALSKRNEKWDEVYKYIVTNWLAKVNTQID
LIRKKMKEALENQAEATKAIINYQYNQYTEEEKNNINFNIDDLSSKLNESINKAMININKFLNQCSVSYLMN
SMIPYGVKRLEDFDASLKDALLKYIYDNRGTLIGQVDRLKDKVNNTLSTDIPFQLSKYVDNQRLLSTFTEYIK
NIINTSILNLRYESNHLIDLSRYASKINIGSKVNFDPIDKNQIQLFNLESSKIEVILKNAIVYNSMYENFSTSFWI
RIPKYFNSISLNNEYTIINCMENNSGWKVSLNYGEIIWTLQDTQEIKQRVVFKYSQMINISDYINRWIFVTIT
NNRLNNSKIYINGRLIDQKPISNLGNIHASNNIMFKLDGCRDTHRYIWIKYFNLFDKELNEKEIKDLYDNQS
NSGILKDFWGDYLQYDKPYYMLNLYDPNKYVDVNNVGIRGYMYLKGPRGSVMTTNIYLNSSLYRGTKFII
KKYASGNKDNIVRNNDRVYINVVVKNKEYRLATNASQAGVEKILSALEIPDVGNLSQVVVMKSKNDQGIT
NKCKMNLQDNNGNDIGFIGFHQFNNIAKLVASNWYNRQIERSSRTLGCSWEFIPVDDGWGERPL

Auch bevorzugt sind Nukleinsäuren, die eine Nukleinsäuresequenz umfassen, die ein Protein mit einer bis zu 80%igen homologen Sequenz, besonders bevorzugt 90%, ganz besonders bevorzugt 95%, zu SEQ ID No 1, SEQ ID NO 2 oder SEQ ID NO 3 kodieren.

Weiterhin bevorzugt ist es, dass die Nukleinsäure der Erfindung die Nukleinsäuresequenz SEQ ID NO 5 umfasst oder zu dieser komplementär ist bzw. die entsprechenden RNA Sequenz. Bevorzugt sind dabei ebenfalls 80% homologe, besonders bevorzugt 90% homologe, ganz besonders bevorzugt 95% homologe Sequenzen.
SEQ ID NO 5:

ATGCCCTTCGTGAACAAGCAGTTCAACTACAAGGACCCCGTGAACGGCGTGGACATCG
CCTACATCAAGATCCCCAACGCCGGCCAGATGCAGCCCGTGAAGGCCTTCAAGATCCA
CAACAAGATCTGGGTGATCCCCGAGCGCGACACCTTCACCAACCCCGAGGAGGGCGA
CCTGAACCCCCCCCCCGAGGCCAAGCAGGTGCCCGTGAGCTACTACGACAGCACCTAC
CTGAGCACCGACAACGAGAAGGACAACTACCTGAAGGGCGTGACCAAGCTGTTCGAGC
GCATCTACAGCACCGACCTGGGCCGCATGCTGCTGACCAGCATCGTGCGCGGCATCCC
CTTCTGGGGCGGCAGCACCATCGACACCGAGCTGAAGGTGATCGACACCAACTGCATC
AACGTGATCCAGCCCGACGGCAGCTACCGCAGCGAGGAGCTGAACCTGGTGATCATCG
GCCCCAGCGCCGACATCATCCAGTTCGAGTGCAAGAGCTTCGGCCACGAGGTGCTGAA
CCTGACCCGCAACGGCTACGGCAGCACCCAGTACATCCGCTTCAGCCCCGACTTCACC
TTCGGCTTCGAGGAGAGCCTGGAGGTGGACACCAACCCCCTGCTGGGCGCCGGCAAG
TTCGCCACCGACCCCGCCGTGACCCTGGCCCACGAGCTGATCCACGCCGGCCACCGC
CTGTACGGCATCGCCATCAACCCCAACCGCGTGTTCAAGGTGAACACCAACGCCTACTA
CGAGATGAGCGGCCTGGAGGTGAGCTTCGAGGAGCTGCGCACCTTCGGCGGCCACGA
CGCCAAGTTCATCGACAGCCTGCAGGAGAACGAGTTCCGCCTGTACTACTACAACAAGT

TCAAGGACATCGCCAGCACCCTGAACAAGGCCAAGAGCATCGTGGGCACCACCGCCAG
CCTGCAGTACATGAAGAACGTGTTCAAGGAGAAGTACCTGCTGAGCGAGGACACCAGC
GGCAAGTTCAGCGTGGACAAGCTGAAGTTCGACAAGCTGTACAAGATGCTGACCGAGA
TCTACACCGAGGACAACTTCGTGAAGTTCTTCAAGGTGCTGAACCGCAAGACCTACCTG
AACTTCGACAAGGCCGTGTTCAAGATCAACATCGTGCCCAAGGTGAACTACACCATCTA
CGACGGCTTCAACCTGCGCAACACCAACCTGGCCGCCAACTTCAACGGCCAGAACACC
GAGATCAACAACATGAACTTCACCAAGCTGAAGAACTTCACCGGCCTGTTCGAGTTCTA
CAAGCTGCTGTGCGTGCGCGGCATCATCACCAGCAAGACCAAGAGCCTGGACAAGGG
CTACAACAAGGCCCTGAACGACCTGTGCATCAAGGTGAACAACTGGGACCTGTTCTTCA
GCCCCAGCGAGGACAACTTCACCAACGACCTGAACAAGGGCGAGGAGATCACCAGCG
ACACCAACATCGAGGCCGCCGAGGAGAACATCAGCCTGGACCTGATCCAGCAGTACTA
CCTGACCTTCAACTTCGACAACGAGCCCGAGAACATCAGCATCGAGAACCTGAGCAGC
GACATCATCGGCCAGCTGGAGCTGATGCCCAACATCGAGCGCTTCCCCAACGGCAAGA
AGTACGAGCTGGACAAGTACACCATGTTCCACTACCTGCGCGCCCAGGAGTTCGAGCA
CGGCAAGAGCCGCATCGCCCTGACCAACAGCGTGAACGAGGCCCTGCTGAACCCCAG
CCGCGTGTACACCTTCTTCAGCAGCGACTACGTGAAGAAGGTGAACAAGGCCACCGAG
GCCGCCATGTTCCTGGGCTGGGTGGAGCAGCTGGTGTACGACTTCACCGACGAGACCA
GCGAGGTGAGCACCACCGACAAGATCGCCGACATCACCATCATCATCCCCTACATCGG
CCCCGCCCTGAACATCGGCAACATGCTGTACAAGGACGACTTCGTGGGCGCCCTGATC
TTCAGCGGCGCCGTGATCCTGCTGGAGTTCATCCCCGAGATCGCCATCCCCGTGCTGG
GCACCTTCGCCCTGGTGAGCTACATCGCCAACAAGGTGCTGACCGTGCAGACCATCGA
CAACGCCCTGAGCAAGCGCAACGAGAAGTGGGACGAGGTGTACAAGTACATCGTGACC
AACTGGCTGGCCAAGGTGAACACCCAGATCGACCTGATCCGCAAGAAGATGAAGGAGG
CCCTGGAGAACCAGGCCGAGGCCACCAAGGCCATCATCAACTACCAGTACAACCAGTA
CACCGAGGAGGAGAAGAACAACATCAACTTCAACATCGACGACCTGAGCAGCAAGCTG
AACGAGAGCATCAACAAGGCCATGATCAACATCAACAAGTTCCTGAACCAGTGCAGCGT
GAGCTACCTGATGAACAGCATGATCCCCTACGGCGTGAAGCGCCTGGAGGACTTCGAC
GCCAGCCTGAAGGACGCCCTGCTGAAGTACATCTACGACAACCGCGGCACCCTGATCG
GCCAGGTGGACCGCCTGAAGGACAAGGTGAACAACACCCTGAGCACCGACATCCCCTT
CCAGCTGAGCAAGTACGTGGACAACCAGCGCCTGCTGAGCACCTTCACCGAGTACATC
AAGAACATCATCAACACCAGCATCCTGAACCTGCGCTACGAGAGCAACCACCTGATCGA
CCTGAGCCGCTACGCCAGCAAGATCAACATCGGCAGCAAGGTGAACTTCGACCCCATC
GACAAGAACCAGATCCAGCTGTTCAACCTGGAGAGCAGCAAGATCGAGGTGATCCTGA
AGAACGCCATCGTGTACAACAGCATGTACGAGAACTTCAGCACCAGCTTCTGGATCCGC
ATCCCCAAGTACTTCAACAGCATCAGCCTGAACAACGAGTACACCATCATCAACTGCAT
GGAGAACAACAGCGGCTGGAAGGTGAGCCTGAACTACGGCGAGATCATCTGGACCCTG
CAGGACACCCAGGAGATCAAGCAGCGCGTGGTGTTCAAGTACAGCCAGATGATCAACA

TCAGCGACTACATCAACCGCTGGATCTTCGTGACCATCACCAACAACCGCCTGAACAAC
AGCAAGATCTACATCAACGGCCGCCTGATCGACCAGAAGCCCATCAGCAACCTGGGCA
ACATCCACGCCAGCAACAACATCATGTTCAAGCTGGACGGCTGCCGCGACACCCACCG
CTACATCTGGATCAAGTACTTCAACCTGTTCGACAAGGAGCTGAACGAGAAGGAGATCA
AGGACCTGTACGACAACCAGAGCAACAGCGGCATCCTGAAGGACTTCTGGGGCGACTA
CCTGCAGTACGACAAGCCCTACTACATGCTGAACCTGTACGACCCCAACAAGTACGTGG
ACGTGAACAACGTGGGCATCCGCGGCTACATGTACCTGAAGGGCCCCCGCGGCAGCG
TGATGACCACCAACATCTACCTGAACAGCAGCCTGTACCGCGGCACCAAGTTCATCATC
AAGAAGTACGCCAGCGGCAACAAGGACAACATCGTGCGCAACAACGACCGCGTGTACA
TCAACGTGGTGGTGAAGAACAAGGAGTACCGCCTGGCCACCAACGCCAGCCAGGCCG
GCGTGGAGAAGATCCTGAGCGCCCTGGAGATCCCCGACGTGGGCAACCTGAGCCAGG
TGGTGGTGATGAAGAGCAAGAACGACCAGGGCATCACCAACAAGTGCAAGATGAACCT
GCAGGACAACAACGGCAACGACATCGGCTTCATCGGCTTCCACCAGTTCAACAACATC
GCCAAGCTGGTGGCCAGCAACTGGTACAACCGCCAGATCGAGCGCAGCAGCCGCACC
CTGGGCTGCAGCTGGGAGTTCATCCCCGTGGACGACGGCTGGGGCGAGCGCCCCCTG

Die vorliegende Erfindung betrifft auch eine pharmazeutische Zusammensetzung, umfassend eine erfindungsgemäße Nukleinsäure und einen pharmazeutisch akzeptablen Träger und/oder Hilfsstoff.

Die Erfindung betrifft außerdem in einer bevorzugten Ausführungsform ein Kit umfassend eine Nukleinsäure gemäß der Erfindung und Hilfsstoffe und ggf. eine Protease.

Bevorzugt ist die pharmazeutische Zusammensetzung zur Applikation in den Muskel oder die Haut oder eine Drüse hergerichtet.

Die anmeldungsgemäße Lehre zeichnet sich vor allem durch die folgenden Merkmale aus:
- Abkehr vom technisch Üblichen
- neue Aufgabenstellung
- Vorliegen eines seit langem ungelösten dringenden Bedürfnisses für die Lösung des mit der Erfindung gelösten Problems
- Entwicklung der wissenschaftlichen Technik ging in eine andere Richtung.

Insbesondere die vorteilhaften Ausführungsformen der Erfindung weisen mindestens einen oder mehrere der genannten Vorteile auf.

### Beispiele

Im Folgenden wir die Erfindung anhand einiger Beispiele uns Figuren illustriert, die jedoch nicht limitierend für die Schutzbereich verstanden werden sollen.

Für unser Ausführungsbeispiel haben wir mRNA des BoNT/A Hall Strains verwendet, die eine Sequenz gemäß SEQ ID No 1 kodiert.. Diese wurde zunächst ohne weitere Zusätze in einer Konzentration von 0,4mg/ml Mäusen intramuskulär in den M. tibialis injiziert. Hierbei hat sich nur eine sehr geringe Paralyse gezeigt (Digit Abduction Score unter 1).

In weiteren Experimenten wurden verschiedene Transfektionsvektoren getestet unter anderem ein polymer-basiertes Transfektionsreagenz (Viromer^{®}) von dem Hersteller Lipocalyx. Dieses wurde entsprechend den Angaben des Herstellers (Lipocalyx) mit der BoNT-mRNA Lösung angesetzt. Hierbei hat man eine deutliche BoNT vermittelte Paralyse feststellen können.
Figur 1:

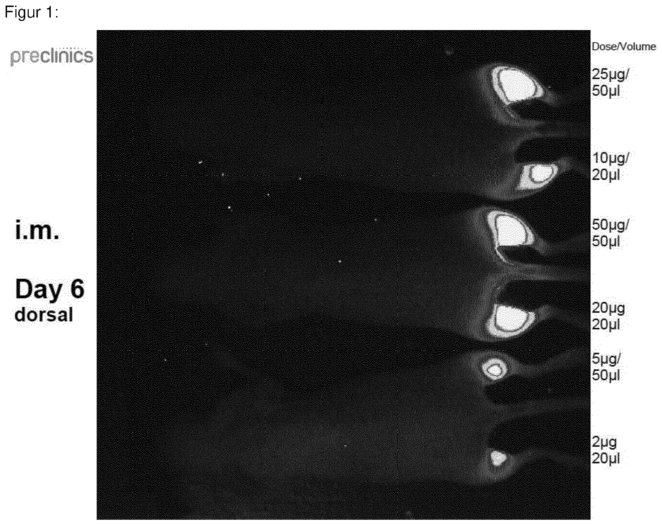

   Figur 1 zeigt eine Biolumineszenz-Aufnahme welche die Luciferase Expression 6 Tage nach Applikation von Plasmid-DNA (pEPI-1-luc) in verschiedenen Dosierungen, Konzentrationen und Volumen zeigt.
Figur 2:

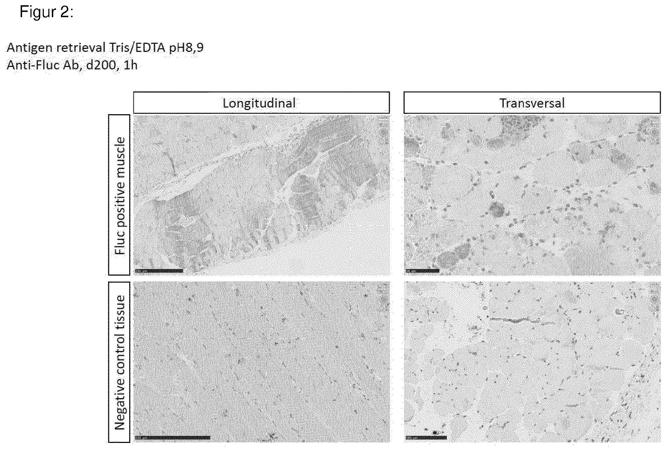

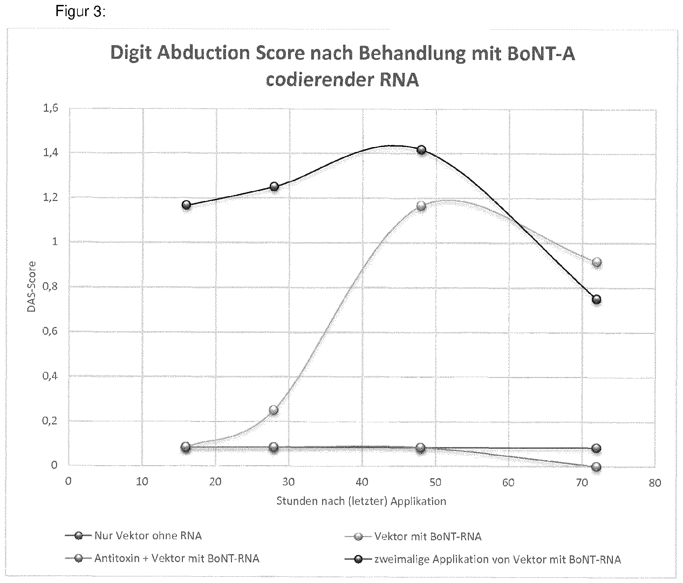

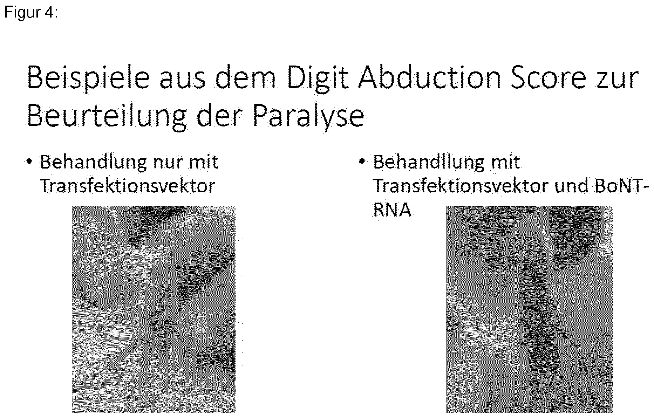

   Figur 2 zeigt eine immunhistologische Färbung von exprimierter Luciferase in Gewebe nach einer Injektion von plasmidDNA in den Muskel (M. gastrocnemius) der Maus. Es ist deutlich zu erkennen, dass sich die Expression auf die Muskelzellen beschränkt und im extrazellulären Raum keine Luciferase zu erkennen ist.
Figuren 3 und 4:
   Der Digit Abduction Score wird verwendet, um in einem Mausmodell die durch BoNT vermittelte Paralyse zu bewerten. Hierbei wird der Zehenspreizreflex der Maus genutzt. Nach einer Injektion in den M. gastrocnemius und oder M. tibialis können die Mäuse je nach Stärke der Paralyse die Zehen nicht oder nur noch eingeschränkt spreizen. Hierbei wird die Paralyse nach einem Bewertungssystem von Aoki et al. Eingeteilt von 0 = keine Paralyse bis 4 komplette Paralyse.
Figur 3:
   In dieser Studie wurden Mäusen in den M. gastrocnemius und den M. tibialis die BoNT-A kodierende RNA mit einem Transfektionsvektor appliziert. In einer Gruppe wurde als Kontrolle nur der Transfektionsvektor appliziert. In einer weiteren Kontrollgruppe wurde ein BoNT-Antitoxin vorab appliziert. Bei den mit BoNT-RNA behandelten Gruppen wurde eine Gruppe nur einmalig behandelt und eine Gruppe erhielt zwei Behandlungen im Abstand von 24h. Beide Kontrollgruppen wiesen keine Paralyse auf, während die behandelten Gruppen zwar keine vollständige, jedoch eine eindeutige Paralyse zeigten.
Figur 4:
   Vergleich einer nicht paralysierten und einer durch BoNT/A-RNA Behandlung paralysierten Hintergliedmaße der Maus.

## Patentansprüche

1. Nukleinsäure zur Verwendung als Arzneimittel und/oder in therapeutischen Verfahren umfassend eine Botulinum Neurotoxin kodierende Nukleinsäure wobei die BoNT kodierende Nukleinsäuresequenz so modifiziert ist, dass das kodierte Protein zwischen leichter und schwerer Kette im Linkerbereich ein Erkennungsmotiv für eine Protease, bevorzugt LVPRGS oder LVPRGS umfasst.

2. Nukleinsäure nach Anspruch 1 zur Behandlung von Spastiken, Dystonien, Speichelauslaufen, Bewegungsstörungen, gestörter Muskelaktivität, Osteosynthese und/oder der Hyperhydrose.

3. Nukleinsäure zur Verwendung/Behandlung nach Anspruch 1 oder 2, wobei die Nukleinsäure eine Sequenz gemäß SEQ ID NO 5 umfasst oder eine Sequenz die dazu 80%, bevorzugt 90% besonders bevorzugt 95% homolog ist.

4. Nukleinsäure zur Verwendung/Behandlung nach einem oder mehreren der vorhergehenden Ansprüche, wobei die Nukleinsäure ein Protein kodiert, das eine der Sequenzen umfasst, ausgewählt aus der Gruppe umfassend SEQ ID NO 1, SEQ ID NO 2, SEQ ID NO 3 und SEQ ID NO 4 oder dazu 80%, bevorzugt 90% besonders bevorzugt 95% homolog ist.

5. Nukleinsäure zur Verwendung/Behandlung nach einem oder mehreren der vorhergehenden Ansprüche zur direkten Applikation in das zu behandelnde Organ und/oder Muskel.

6. Nukleinsäure zur Verwendung/Behandlung nach einem oder mehreren der vorhergehenden Ansprüche zur Transfektion von Skelettmuskelzellen, glatten Muskelzellen, glatten Muskelzellen der Drüsen oder der Haut und/oder anderer Hautzellen.

7. Nukleinsäure zur Verwendung/Behandlung nach einem oder mehreren der vorhergehenden Ansprüche, wobei es sich um eine DNA oder RNA handelt.

8. Nukleinsäure zur Verwendung/Behandlung nach einem oder mehreren der vorhergehenden Ansprüche, wobei die Nukleinsäuresequenz, die BoNT kodiert, BoNT/A, BoNT/B, BoNT/C, BoNT/D, BoNT/E BoNT/F oder BoNT/G kodiert.

9. Nukleinsäure zur Verwendung/Behandlung nach einem oder mehreren der vorhergehenden Ansprüche zusätzlich umfassend eine Sequenz, die ein sekretorisches Signal kodiert.

10. Nukleinsäure zur Verwendung/Behandlung nach einem oder mehreren der vorhergehenden Ansprüche zusätzlich umfassend ein S/MAR Element.

11. Nukleinsäure zur Verwendung/Behandlung nach einem oder mehreren der vorhergehenden Ansprüche zusätzlich umfassend eine Sequenz welche die erforderliche Protease kodiert.

12. Verwendung von einer Nukleinsäure die eine Botulinum Neurotoxin kodierende Nukleinsäure umfasst zu nicht-therapeutischen, kosmetischen Zwecken, wobei die Nukleinsäure eine der Nukleinsäuren nach Anspruch 1 bis 11 ist.

13. Verwendung nach Anspruch 12, wobei das in vivo exprimierte Botulinum Neurotoxin durch eine gleichzeitig oder nachträglich injizierte Protease aktiviert wird.

14. Pharmazeutische Zusammensetzung, umfassend eine Nukleinsäure gemäß den Ansprüchen 1 bis 11 und einen pharmazeutisch akzeptablen Träger und/oder Hilfsstoff.

## Claims

1. A nucleic acid for use as a drug and/or in therapeutic methods comprising a botulinum neurotoxin-encoding nucleic acid, wherein the BoNT-encoding nucleic acid sequence is modified such that the encoded protein between the light and heavy chains in the linker region comprises a recognition motif for the respective protease, preferably LVPRGS or LVPRGS.

2. The nucleic acid according to claim 1 for the treatment of spasticity, dystonia, saliva leakage, movement disorders, impaired muscle activity, osteosynthesis and/or hyperhydrosis.

3. The nucleic acid for use/treatment according to claim 1 or 2, wherein the nucleic acid comprises a sequence according to SEQ ID NO 5 or a sequence 80%, preferably 90%, particularly preferred 95% homologous thereto.

4. The nucleic acid for use/treatment according to any one or more of the preceding claims, wherein the nucleic acid encodes a protein which comprises one of the sequences selected from the group comprising SEQ ID NO 1, SEQ ID NO 2, SEQ ID NO 3 and SEQ ID NO 4, or 80%, preferably 90%, particularly preferred 95% homologous thereto.

5. The nucleic acid for use/treatment according to any one or more of the preceding claims for direct application in the organ to be treated and/or muscle.

6. The nucleic acid for use/treatment according to any one or more of the preceding claims for the transfection of skeletal muscle cells, smooth muscle cells, smooth muscle cells of the glands or the skin and/or other skin cells.

7. The nucleic acid for use/treatment according to any one or more of the preceding claims, wherein said nucleic acid is a DNA or RNA.

8. The nucleic acid for use/treatment according to any one or more of the preceding claims, wherein the BoNT-encoding nucleic acid sequence encodes BoNT/A, BoNT/B, BoNT/C, BoNT/D, BoNT/E, BoNT/F or BoNT/G.

9. The nucleic acid for use/treatment according to any one or more of the preceding claims additionally comprising a secretory signal-encoding sequence.

10. The nucleic acid for use/treatment according to any one or more of the preceding claims additionally comprising a S/MAR element.

11. The nucleic acid for use/treatment according to any one or more of the preceding claims additionally comprising a necessary protease-encoding sequence.

12. A use of a nucleic acid comprising a botulinum neurotoxin-encoding nucleic acid for non-therapeutic cosmetic purposes, wherein the nucleic acid is one of the nucleic acids according to claims 1 to 11.

13. The use of claim 12, wherein said botulinum neurotoxin expressed in vivo is activated by a simultaneously or subsequently injected protease.

14. A pharmaceutical composition comprising a nucleic acid according to claims 1 to 11 and a pharmaceutically acceptable carrier and/or excipient.

## Revendications

1. Acide nucléique destiné à être utilisé comme médicament et/ou dans des procédés thérapeutiques comprenant un acide nucléique codant pour la neurotoxine botulique,
dans lequel la séquence d'acide nucléique codant BoNT est modifiée de telle sorte que la protéine codée entre une chaîne légère et une chaîne lourde dans la région de liaison comprend un motif de reconnaissance pour une protéase, de préférence LVPRGS ou LVPRGS.

2. Acide nucléique selon la revendication 1 pour le traitement des spasmes, des dystonies, des écoulements salivaires, des troubles du mouvement, des activité musculaire perturbée, de l'ostéosynthèse et/ou de l'hyperhydrose.

3. Acide nucléique pour l'utilisation/le traitement selon la revendication 1 ou 2, dans lequel l'acide nucléique comprend une séquence de SEQ ID N° 5 ou une séquence homologue à celle-ci à 80 %, de préférence à 90 %, de manière particulièrement préférée à 95 %.

4. Acide nucléique pour l'utilisation/le traitement selon l'une ou plusieurs des revendications précédentes, dans lequel l'acide nucléique code pour une protéine comprenant l'une des séquences choisies dans le groupe comprenant SEQ ID N° 1, SEQ ID N° 2, SEQ ID N° 3 et SEQ ID N° 4, ou est homologue à 80 %, de préférence à 90 %, de manière particulièrement préférée à 95 %.

5. Acide nucléique pour l'utilisation/le traitement selon l'une ou plusieurs des revendications précédentes, pour une application directe dans l'organe et/ou le muscle à traiter.

6. Acide nucléique pour l'utilisation/le traitement selon l'une ou plusieurs des revendications précédentes pour la transfection de cellules musculaires squelettiques, de cellules musculaires lisses, de cellules musculaires lisses de glandes ou de la peau et/ou d'autres cellules de la peau.

7. Acide nucléique pour l'utilisation/le traitement selon l'une ou plusieurs des revendications précédentes, dans lequel il s'agit d'un ADN ou d'un ARN.

8. Acide nucléique pour l'utilisation/le traitement selon l'une ou plusieurs des revendications précédentes, dans lequel la séquence d'acide nucléique codant BoNT code BoNT/A, BoNT/B, BoNT/C, BoNT/D, BoNT/E, BoNT/F ou BoNT/G.

9. Acide nucléique pour l'utilisation/le traitement selon l'une ou plusieurs des revendications précédentes, comprenant en outre une séquence codant un signal sécrétoire.

10. Acide nucléique pour l'utilisation/le traitement selon l'une ou plusieurs des revendications précédentes, comprenant en outre un élément S/MAR.

11. Acide nucléique pour l'utilisation/le traitement selon l'une ou plusieurs des revendications précédentes, comprenant en outre une séquence codant la protéase requise.

12. Utilisation d'un acide nucléique comprenant un acide nucléique codant une neurotoxine botulique à des fins cosmétiques, non thérapeutiques, dans lequel l'acide nucléique est l'un des acides nucléiques selon les revendications 1 à 11.

13. Utilisation selon la revendication 12, dans lequel la neurotoxine botulique exprimée in vivo est activée par une protéase injectée simultanément ou ultérieurement.

14. Composition pharmaceutique comprenant un acide nucléique selon les revendications 1 à 11 et un support et/ou un excipient pharmaceutiquement acceptable.
